# EUROPEAN PATENT SPECIFICATION

(11) **EP 2 385 820 B1**
(45) Date of publication and mention of the grant of the patent: **12.04.2017**
(21) Application number: 09777659.5
(22) Date of filing: 05.08.2009
(51) Int. Cl.: A61K 8/98, A61Q 19/00, A61Q 19/02, A61Q 19/08, A61K 8/365

(54) **COSMETIC USE OF A CORAL POWDER**
KOSMETISCHE VERWENDUNG EINES KORALLENPULVERS
USAGE COSMÉTIQUE DE POUDRE DE CORAIL

(30) Priority: 12.01.2009 IT MI20090018
(43) Date of publication of application: 16.11.2011
(73) Proprietor: Kalichem S.r.l., 25086 Rezatto (BS) (IT)
(72) Inventor: Capelli, Cristina, 16146 Genova (IT)
(74) Representative: Ferreccio, Rinaldo
(86) International application number: PCT/EP2009/005656
(87) International publication number: WO 2010/078879

(56) References cited:
- EP-A- 0 115 216
- EP-A2- 0 508 324
- GB-A- 1 260 332
- JP-A- 61 212 508
- RO-B1- 117 897
- RO-B1- 121 765
- "Herbal skin care:Shankara facial cleanser" AYUR BALANCE: SHOP FOR AYURVEDIC PRODUCTS, [Online] 5 February 2005 (2005-02-05), XP002541878 Retrieved from the Internet: URL:http://web.archive.org/web/20050205085 524/http://www.ayurbalance.com/shop_shankt rifacecleanser.htm> [retrieved on 2009-08-18]
- DATABASE CA [Online] CHEMICAL ABSTRACTS SERVICE, COLUMBUS, OHIO, US; 27 December 2000 (2000-12-27), NAGAHATA, TETSUJI: "Cosmetics containing coral powder, colloidal hydrated silicates, and benzoate salts" XP002541879 retrieved from STN Database accession no. 2000:907023 -& JP 2000 355515 A (KANSAI KOSO K. K., JAPAN) 26 December 2000 (2000-12-26)
- DATABASE WPI Week 199718 Thomson Scientific, London, GB; AN 1997-197191 XP002541880 "UV-light interrupting agent for cosmetic and absorbing sebum or sweat - comprises natural coral powder" -& JP 9 052815 A (MARINEBIO KK) 25 February 1997 (1997-02-25)
- DATABASE WPI Week 200038 Thomson Scientific, London, GB; AN 2000-442648 XP002541881 "Ultraviolet shielding agents comprising coral sand powder and wild silkworm cocoon shell powder, are for use for cosmetic preparations" -& WO 00/37581 A (MARINE BIO CO LTD) 29 June 2000 (2000-06-29)
- "Abrasive compsn. used in dentifrice - comprises porous coral powder of specified particle size and fine pore dia" DERWENT HOST- DERWENT, 1 July 1991 (1991-07-01), XP002265099
- JELENA CVEJIC ET AL: "Determination of canthaxanthin in the red coral (Corallium rubrum) from Marseille by HPLC combined with UV and MS detection", MARINE BIOLOGY ; INTERNATIONAL JOURNAL ON LIFE IN OCEANS AND COASTAL WATERS, SPRINGER, BERLIN, DE, vol. 152, no. 4, 28 June 2007 (2007-06-28) , pages 855-862, XP019538117, ISSN: 1432-1793, DOI: 10.1007/S00227-007-0738-5

## Description

### State of the art

The present invention relates to the state of the art of the cosmetics industry. More particularly the invention relates to the use in cosmetics of a particular type of coral and to cosmetic formulations which contain it.

### Background art

With the increase in phenomena of hypersensitivity linked to the use of organic materials of synthetic origin or derived from animal sources, research in the area of cosmetology has aimed at identifying natural materials able to replace these potentially allergenic organic materials

One example of a material of natural origin which has been proposed as the ingredient of creams, face packs, exfoliant scrubs and make-up, etc. is the Coral Calcium powder of Natural Sourcing LLC (U.S.A.).

This powder is obtained from the coral deposited on the beaches of the island of Okinawa and basically consists of calcium carbonate to which a series of trace elements add, including magnesium, sulphur, silicon, strontium, boron, copper, fluorine, iodine, manganese, molybdenum, phosphorus, zinc, rubidium, selenium and vanadium. Nevertheless heavy metals such as arsenic and lead are also present, albeit in such stated quantities as not to create problems of toxicity.

The Coral Calcium powder is white in colour and has a density of 2.83 g/cm³ and a particle size of 20/40 µm.

The research carried out by the Applicant has led to the identification of a new source of materials suitable for use in the cosmetic sector; this new source consists of red coral, *Corallium rubrum*, which is an octocoral of the family of the Corallidae and is the only species of the *Corallium* genus which lives in the Mediterranean.

The calcareous skeleton of red coral is highly sought after as a material for the production of jewellery of high value. Currently red coral is fished in Red coral, albeit very hard, is also somewhat fragile and difficult to work and a great deal of scrap is generated during processing.

"Herbal skin care: Shankara facial cleanser", Ayur Balance: Shop for ayurvedic products, [Online] 5 February 2005, XP002541878, discloses a facial cleanser composition based on natural ingredients, to be used as daily cleanser or as a nourishing mask, ahving a deep-cleansing, exfoliating, purifying and nourishing action on the skin. This composition contains 27 ingredients, among which red coral powder, which is attributed deeply nurturing, complexion enhancing quality. Nothing is said about the particle size of the red coral powder contained in this composition.

JP 09 052815 A discloses cosmetic compositions comprising a UV-shielding agent, consisting of coral powder obtained by crushing coral sand.

WO 00/37581 relates to UV shielding agents containing an optionally decarboxylated coral sand powder and a wild silkworm cocoon shell powder.

One of the objects of the present invention is that of making available a powder of natural origin suitable for being incorporated in cosmetic formulations without any risk of a toxicological and/or allergological nature.

### Summary of the invention

Such an object has been achieved by a cosmetic formulation according to claim 1, comprising a powder obtained from the milling of red coral (*Corallium rubrum*), in particular from the scrap produced by the working of red coral by jewellers.

More particularly the powder according to the present invention has a particle size of about 4-20 µm, when it has to be used in cosmetic preparations for skin care, make-up (purely by way of an example we can mention eye shadow, blusher, rouge, powder, foundation and lipstick) and tanning products. It has preferably a particle size of about 4-6 µm, preferably 4 to less than 5 µm, when an anti-ageing function is also required, due to its action of filling, texturising, evening out of the complexion with fading of minor skin imperfections, smoothing, mattifying and SPF (sun protection factor) boosting. Sizes between 8 and 20 µm can be recommended for the above-mentioned cosmetic preparation, when a soft focus, illuminating effect is sought.

The cosmetic formulations according to the present invention generally contain 0.1% to 100% red coral powder by weight out of the total weight of the formulation. In particular, they contain 95% to 100% red coral powder by weight out of the total weight of the formulation, when they are to be used as a face powder or blush. In such a case, they may contain a minor amount of one or more further powdery components, such as e.g. lubricating agents.

When the formulations according to the present invention contain a cosmetically acceptable vehicle and optionally at least one further cosmetically active ingredient, then red coral powder is contained in an amount of about 0.1% to 10%, preferably 0.25% to 2%, by weight out of the total weight of the formulation.

The cosmetic formulations according to the present invention may also contain at least one weak acid, in particular an acid selected from among lower carboxylic acids and phosphoric acid, and advantageously an acid selected among citric, lactic, tartaric, and gluconic acid so that the pH of the formulation is ≤7, preferably between 6.8 and 5.

Lactic acid and gluconic acid are particularly preferred as the weak acids to be added to the formulation according to the present invention and either one of them is generally contained in an amount of about 0.01 to 10% by weight out of the weight of the formulation. Through the controlled addition of a weak acid, a certain amount of CO₂ develops from the red coral powder, what gives rise to a foamy consistency of the formulation, particularly when the latter is in the form of a cream, and to an increased bioavailability of the oligoelements included in the red coral powder.

The red coral powder is preferably contained inside the above-mentioned cosmetic formulations in a quantity, as a weight percentage of the total weight of the formulation, of between 0.1% and 100%. The red coral powder according to the invention can, in fact, also be used as-is, for application using a brush like a face powder or blush. The face powder or blush may contain a reduced amount of other powdery substances, up to 5% by weight of the total weight of the powder.

For cosmetic applications other than as face powder or blush, the red coral powder is preferably contained in an amount of 0.1% to10%, advantageously between 0.25% and 2%, as a weight percentage of the total weight of the formulation.

In another aspect the invention relates to the use of red coral powder (*Corallium rubrum*) as a cosmetically active substance, according to claim 9. In one of its additional aspects the present invention relates to a method of cosmetic treatment according to claim 10.

When the red coral powder is part of a cosmetic formulation including a cosmetically acceptable vehicle and optional further cosmetically active substances, the addition of preservatives may be required or convenient. In such cases, the usual preservatives used for cosmetic preparations can be utilized, with the following being particularly preferred: ISOCIDE BAS (Benzyl Alcohol, Dehydroacetic Acid, Aqua) for creams, and PHENOXYETHANOL for exfoliating gels and powders. In the examples provided in the detailed description of the invention, the two preservatives mentioned above have been used, as appropriate for each kind of formulation.

The powder according to the present invention constitutes a precious basic material for the preparation of various cosmetic formulations such as, for example, natural mineral preparations for skin care, natural mineral make-up and natural mineral tanning products, to which it confers useful properties such as radiance, improvement in the skin texture, filler effect, matte effect, SPF (sun protection factor) booster effect and covering effect.

It is to be remarked that the products with filler effects (in particular those used for minimizing wrinkles and skin defects), which are currently available on the market are of synthetic origin, whereas the powder according to the present invention is wholly natural. Moreover, in addition to this filling effect, the powder of the invention and the formulation containing it simultaneously display also a soft-focus effect. The latter effect is not normally displayed by the currently available products with filling effect.

The powder according to the invention can also constitute an ingredient for natural mineral products for personal care or hair care, such as for example shampoos, conditioners, packs for hair, bath foams, lotions, etc.

Moreover the presence of important oligoelements in the powder according to the invention, in particular iron, copper and zinc, provides it with the characteristics of an anti-ageing agent.

The powder according to the invention has likewise demonstrated that it has properties of self-preservation and therefore does not need the addition of preservatives.

Then it should be underlined that the powder according to the present invention is obtained exclusively from the scrap from the working of red coral, in order to safeguard the species *Corallium rubrum* and in full respect for the marine environment.

Finally, it is worth mentioning that red coral is obtained from Mediterranean sea, which is substantially radioactivity-free, differently from the Japan sea, from which the known Coral Calcium powder is obtained.

### Brief description of the drawings

Fig. 1 is a photo at the scanning electron microscope of a sample of the powder according to the invention.
Fig. 2 is an ellipsometry graph showing the index of refraction of a sample of the powder according to the invention.
Fig. 3 includes the XRPM diffractograms of two samples of the powders obtained from Examples 1 and 2.

### Detailed description

By way of an example the following is a description of preparation of a powder according to the invention with particle size smaller than 5 µm.

### Example 1

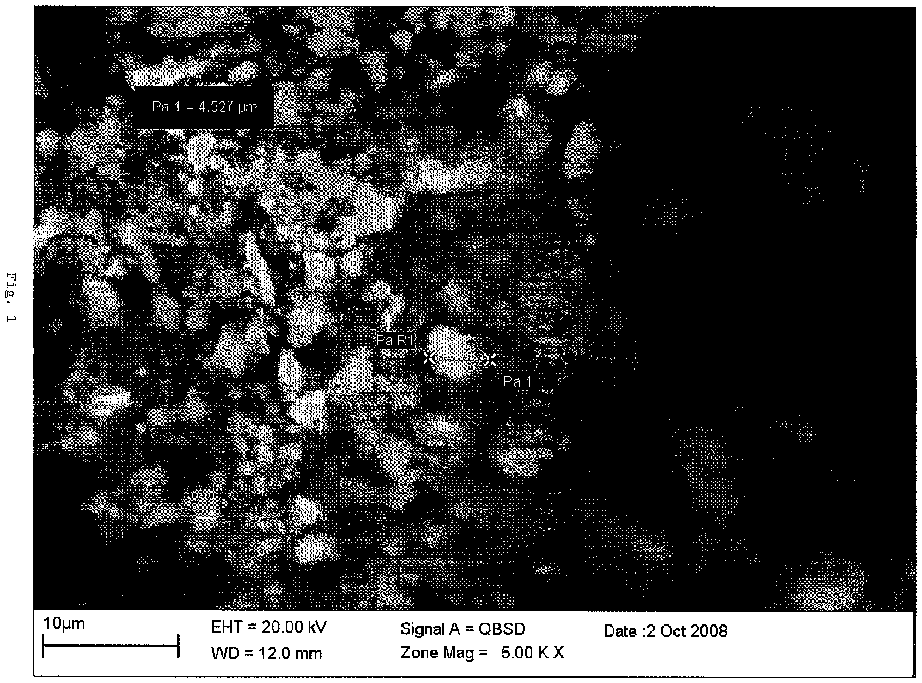

100 g of scrap from the working of red coral from Sardinia (Italy) were subjected to milling in a tungsten ball mill at 300 rpm for 10 minutes, followed by a 10-minute pause, another 10-minute milling, another 10-minute pause and so forth, until milling was carried out for a total period of 90 minutes. The balls/coral weight ratio was 3.5/1. It was thus obtained, with a yield of about 82%, after sieving with a 45 micrometre sieve, a pink powder with particles of average size below 5 µm (see the SEM photo of Fig. 1).
The powder fraction that did not pass through the 45 micrometer sieve showed a more intense pink color and a particle size comprised between 100 and 200 micrometer, as assessed through scanning electron microscope photos. However, from photos taken with a larger magnification it was noticed that such particles are in fact aggregates of smaller particles.
This fraction can advantageoulsy be used for preparing an extract containing water, glycerin, propanediol and an appropriate buffer to maintain pH in a range between 4 and 7.

An analysis performed with the microprobe of the scanning electron microscope revealed the presence of the main ingredient, calcium carbonate, and of Mg, Na and Cl.

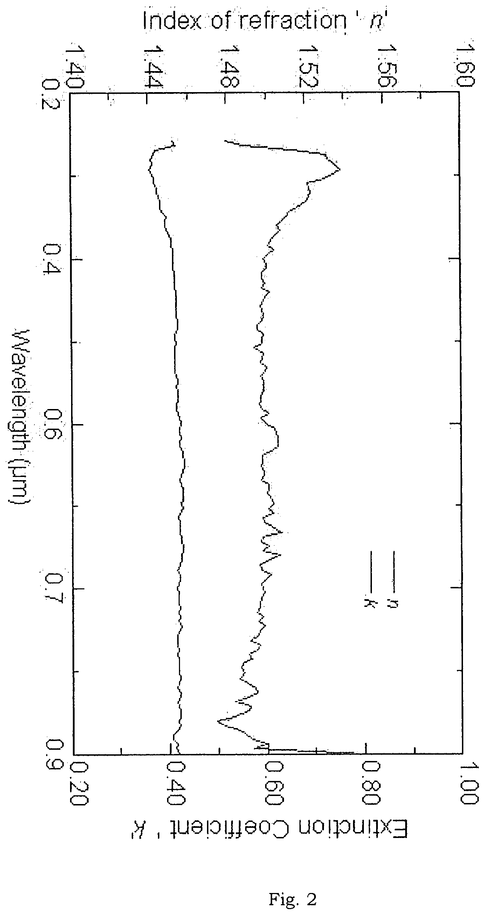

By means of X ray diffraction it was ascertained that the crystalline structure of the powder corresponds to that of calcite, the rombohedral allotropic form of calcium carbonate.
The surface area of the powder fraction which passed through the 45 micrometer sieve was determined through the BET method and it resulted to be equal to 14.27 m²/g.This value is higher than the BET surface area values of other fine cosmetic powders used for filling wrinkles, hiding imperfection and promoting radiance, such as e.g. silica powder, which has a BET surface area of 9 m²/g.
The same powder fraction was analyzed by ellipsometry, to determine its optical properties. As it clearly appears from Figure 2, the refraction index determined by ellipsometry is around 1.5, which is quite close to the refraction index of glass (1.4) and crystal (1.56).

The content of trace elements present in the powder according to the invention was also determined, by atomic absorption, obtaining the results summarised in Table 1 below.

**TABLE 1**

| | Pb | Cd | Ni | Cu | Zn | Co | Fe | Mn | Ag |
|---|---|---|---|---|---|---|---|---|---|
| µg/g | <0.1 | <0.01 | none | 2.44 | 6.5 | 0.16 | 38.7 | 3.24 | 1.15 |

It should be remembered that the known Coral Calcium product contains detectable quantities of heavy metals, in particular lead, whereas the powder according to the invention reveals the substantial absence of lead and nickel.

It is also noted that the powder according to the invention contains a significant quantity of iron which is not instead contained in appreciable quantities in the Coral Calcium product. The iron contributes a great deal to the particular colouring of the powder according to the invention and also has an effect of anti-ageing and protection against the early skin ageing caused by UV rays.

Iron in fact intervenes in many enzymatic reactions in cells and facilitates the action of catalases having iron as co-factor which have an important function in the fight against free radicals. Iron also participates in the metabolic activity of the nucleus of the cell, in the synthesis of DNA and the reactions of energy metabolism. More particularly iron, together with vitamin C, is a vital factor in the re-activation of the synthesis of pro-collagen and consequently re-establishes the tone and resistance of mature skin.

It is also considered that the actual high content of iron is responsible for the illuminating effect and the effect of covering skin imperfections that are typical of cosmetic formulations containing the powder according to the present invention.

It should also be noted that, although the powder according to the invention is subjected to the appropriate treatments of sanitisation laid down by the rules of good manufacture before its incorporation in the various cosmetic formulations, in actual fact it already has from the start an extremely low microbe content, despite the fact it comes from the scrap produced by working coral which is performed in environments which do not require aseptic conditions and not even observance of particular rules of hygiene, such as in the case for example of the processing of food.

This demonstrates that the powder according to the present invention has unexpected characteristics of self-preservation.

Finally it has been found that the powder according to the present invention and the cosmetic formulations which contain it have outstanding characteristics of reflection of UV rays and consequently it is supposed to protect the skin against the harmful effects of solar radiation.

### Example 2

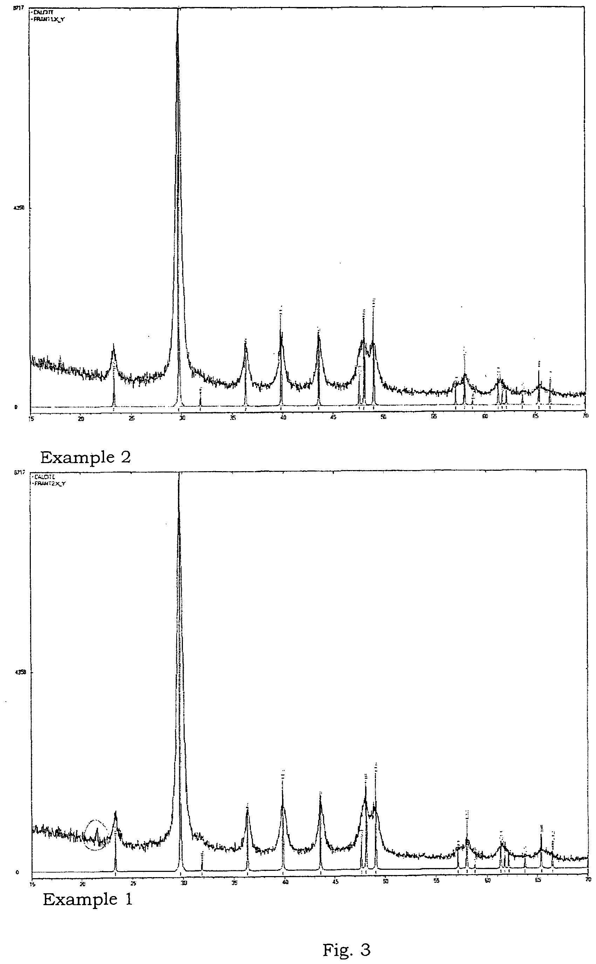

100 g of scrap from the working of red coral from Campania (Italy) were subjected to milling in a tungsten ball mill exactly in the same conditions as for Example 1. It was obtained, with a yield of about 81%, after sieving with a 45 micrometre sieve, a pink powder with particles of average size below 5 µm, as determined by SEM.
This powder was subjected to XRPM analysis in comparison with the powder with particle size below 5 µm obtained in Example 1, in order to investigate possible structural differences according to geographical origin. The diffractograms obtained are shown in Figure 3 and confirm that Corallium Rubrum is mainly composed of calcite, which is the rhombohedral allotropic form of CaCO₃ and that there are no significant structural differences according to geographical origin of Corallium Rubrum.

### Example 3

With the powder with particle size lower than 5 µm obtained according to example 1 the following formulation was prepared in the form of a cream.

**FACE CREAM**

| SUPPLIER | INGREDIENT | INCI NAME | % |
|---|---|---|---|
| GATTEFOSSE | EMULIUM KAPPA | 1 | 6.00 |
| GATTEFOSSE | COMPRITOL 888 | 2 | 2.50 |
| COGNIS | CETIOL PGL | 3 | 3.00 |
| PCP | DERMOFEEL MCT | TRICAPRYLIN | 2.00 |
| COGNIS | CEGESOFT PFO | PASSIFLORA INCARNATA SEED OIL | 1.50 |
| COGNIS | CETIOL LDO | 4 | 3.50 |
| AKOTT | EUXYL K 702 (KONSER 702) | 5 | 0.60 |
| PCP | DERMOFEEL TOCO 70 *NON-GMO* | 6 | 0.030 |
| | PURIFIED WATER | AQUA (WATER) | 70.97 |
| AKOTT | VEGETABLE GLYCERINE | GLYCERIN | 5.00 |
| BIOCHIM | AVICEL PC 611 | 7 | 2.300 |
| ACEF | KELTROL | XANTHAN GUM | 0.30 |
| | RED CORAL | CORAL POWDER | 2.00 |
| SFA | MARIN JASMIN 43111-24111604 | PARFUM/FRAGRANCE | 0.30 |
| | | | 100.00 |

1 = CANDELILLA/JOJOBA/RICE BRAN POLYGLYCERYL-3 ESTERS, GLYCERYL STEARATE, CETEARYL ALCOHOL, SODIUM STEAROYL LACTYLATE
2 = GLYCERYL DIBEHENATE, TRIBEHENIN, GLYCERYL BEHENATE
3 = HEXYLDECANOL, HEXYLDECYL LAURATE
4 = DICAPRYLYL ETHER, LAURYL ALCOHOL
5 = PHENOXYETHANOL, DEHYDROACETIC ACID, BENZOIC ACID
6 = TOCOPHEROL, HELIANTHUS ANNUUS (SUNFLOWER) SEED OIL
7 = MICROCRYSTALLINE CELLULOSE, CELLULOSE GUM

The percentages given above are in weight out of the total weight of the formulation.

The face cream was prepared as follows.

The first eight ingredients in the table given above were mixed and heated to 70°C. The purified water and the vegetable glycerine were mixed and heated separately to 80°C and Avicel PC 611 and Keltrol were dispersed in this mixture. The mixture obtained in this way was added to the mixture of the first eight ingredients and all this was emulsified.

The red coral powder of example 1 (more precisely, the fraction thereof having a particle size below 5 µm) and the Marin Jasmin 43111-24111604 fragrance were added while stirring to the emulsion obtained, brought to the temperature of 40°C, continuing cooling until reaching room temperature.

The cream obtained in this way was found to be perfectly stable both from the physical viewpoint (no separation of phases) and from the microbiological viewpoint (no development of micro-organisms).

This cream was also subjected to sensory evaluation in a blind study. Thirty female volunteers with different types of skin and aged 30-65 were asked to apply the cream of Example 3 twice daily for 1 month. The experimental protocol envisaged the evaluation of 5 selected parameters.

The results of this study are summarized in the following Table 2.

**TABLE 2**

| Parameter | Radiance | Improvement of relief | Mattifying - sensory effect | Firmness | Filler effect |
|---|---|---|---|---|---|
| Volunteers | Enhances the radiance and promotes a fresh and healthier look | Smoothes the skin, refines the texture, attenuates fine lines and wrinkles | Reduces shine with a powdery and velvety feel | Improves tone and elasticity | The skin looks redensified, more compact |
| % | 70% | 65% | 68% | 71% | 73% |

100% panelists observed a visible improvement in the appearance of the skin, from the second day of application with a very high level of satisfaction.

The fraction of the red coral powder accordig to claim 1 with a particle size below 5 µm was also used to preparare the formulations of the following examples

### Example 4

**NATURAL REGENERATING FACE CREAM**

| SUPPLIER | INGREDIENT | INCI NAME | % |
|---|---|---|---|
| PHASE A | | | |
| GATTEFOSSE | EMULIUM KAPPA | 1 | 6.00 |
| GATTEFOSSE | COMPRITOL 888 | 2 | 3.00 |
| COGNIS | CETIOL PGL | 3 | 1.50 |
| PCP | DERMOFEEL MCT | TRICAPRYLIN | 3.50 |
| COGNIS | CETIOL LDO | 4 | 3.00 |
| AKOTT | EUXYL K 702 (KONSER 702) | 5 | 0.60 |
| PCP | DERMOFEEL TOCO 70 *NON-GMO* | 6 | 0.030 |

| PHASE B | | | |
|---|---|---|---|
| | PURIFIED WATER | AQUA (WATER) | Q.S. to 100 |
| AKOTT | ZEMEA PROPANEDIOL | | 4.50 |
| AKOTT | BIOCOL AX | 7 | 1.0 |
| ACEF | KELTROL | XANTHAN GUM | 0.10 |
| FASE C | | | |
| | RED CORAL | CORAL POWDER | 2.00 |
| | | PARFUM/FRAGRANCE | 0.30 |
| ACEF | LACTIC ACID | LACTIC ACID | Q.S. to pH 5.8 |
| | | | 100.00 |

1 = CANDELILLA/JOJOBA/RICE BRAN POLYGLYCERYL-3 ESTERS, GLYCERYL STEARATE, CETEARYL ALCOHOL, SODIUM STEAROYL LACTYLATE
2 = GLYCERYL DIBEHENATE, TRIBEHENIN, GLYCERYL BEHENATE
3 = HEXYLDECANOL, HEXYLDECYL LAURATE
4 = DICAPRYLYL ETHER, LAURYL ALCOHOL
5 = PHENOXYETHANOL, DEHYDROACETIC ACID, BENZOIC ACID
6 = TOCOPHEROL, HELIANTHUS ANNUUS (SUNFLOWER) SEED OIL
7 = MICROCRYSTALLINE CELLULOSE, CELLULOSE GUM

All the components of phase A but DERMOFEEL TOCO 70 are heated to 75°C.

BIOCOL AX and KELTROL are thoroughly dispersed into a mixture of ZEMEA and AQUA that had previously been heated to 75°C..

Phase A is added to phase B in a turbo-mixer, while carefully checking that temperature is constantly kept at 70°C under the action of the turbine for at least 10 minutes. Then the turbine is stopped and DERMOFEEL TOCO 70 is added under fast stirring and afterwards cooling is started.

When a temperature of 40°C has been reached, RED CORAL is added under slow stirring and stirring is continued until room temperature is reached. Finally the pH is adjusted by adding lactic acid.

### Example 5

**RESTITUTIVE ANTI-AGEING FACE CREAM**

| **PHASE A** | | |
|---|---|---|
| EMULPROGRESS 57 | Polygryceryl-10 Stearate (and) Polyglyceryl-6 Tristearate (and) Cetearyl Alcohol (and) Hydroxypropyl Guar | 6.00 |
| BIOPHYTOSEBUM | Decyl Olive Esters (and) Squalene | 5.00 |
| PHYTOSQUALAN | Squalene | 3.00 |
| OLIVE CERESTERS | Decyl Olivate | 2.00 |
| PRESERVATIVE | | Q.S. |
| DERMOFEEL TOCO 70 | Tocopherol (and) Helianthuus Annus (sunflower) Seed Oil | 0.05 |
| PHYTOWAX OLIVE 18L57 | Hydrogenated Stearyl Olive Esters | 1.50 |

| **PHASE B** | | |
|---|---|---|
| WATER | AQUA | Q.S. to 100 |
| ZEMEA | Propanediol | 5.00 |
| BIOCOL AX | Pectin (and) Xanthan gum | 1.00 |

| **PHASE C** | | |
|---|---|---|
| SENSHYAL | Hyaluronic Acid (and) Rhizobian Gum | 2.00 |
| OLIGOGELINE | Condrus Crispus (Carraggenan) | 2.00 |
| HOMEO-AGE | Ascophyllum Nodosum Extract (and) Sorbitol (and) Water | 1.50 |
| HOMEOXY | Sorbitol (and) Enteromorpha Compressa Extract (and) Palmaria Palmata Extract (and) Water | 1.00 |
| CHRONOLINE | Glycerin (and) Water (and) Dextran (and) Caprooyl-Tetrapeptide-3 | 1.00 |
| KOLLAREN | Water (and) Dextran (and) Tripeptide-1 | 3.50 |
| HOMEO-SHIELD | Fucus Serratus Extract (and) Glycerin | 1.00 |
| HYDRIAME | Water (and) Glycosaminoglycans (and) Sclerotium Gum (and) Chitosan Glycolate (and) Sodium Hydroxide | 3.00 |
| LANABLUE | Sorbitol (and) Water (and) Algae Extract | 2.50 |
| NEUTRAZEN | Water (and) Butylene glycol (and) Dextran (and) Palmitoyl Tripeptide-8 | 1.00 |
| EXO T | Butylene Glycol (and) Vibrio Alginolyticus Ferment Filtrate | 0.50 |
| EXO H | Butylene Glycol (and) Alteromonas Ferment Filtrate | 1.00 |
| RED CORAL | Coral Powder | 2.00 |
| TRANSCUTOL CG | Ethoxydiglycol | 1.20 |
| KSG-15 | Dimethicone/Vinyldimethicone Crosspolymer | 1.50 |
| FRAGRANCE | Parfum | Q.S |
| LACTIC ACID | Lactic Acid | Q.S. to pH 5.8 |

This cream is prepared as follows.

The ingredients listed under Phase A are mixed and heated to 75°C.

Phase B is prepared by heating propanediol and water and by dispersing thereinto BIOCOL AX.

Phase A is added to Phase B and the resulting mixture is stirred in a turbo-mixer for 10 minutes. Afterwards, the mixture is cooled to 40°C under fast stirring. Once this temperature has been reached, all the components listed under Phase C, but lactic acid, are added one after the other to the above mixture and the final mixture is brought under slow stirring down to room temperature. Finally, lactic acid is added in the required amount to adjust the pH to 5.8.

### Example 6

**ANTI-AGEING ENLIGHTENING FACE CREAM**

| **PHASE A** | | |
|---|---|---|
| EMULIUM 22 | Tribehenin PEG-20 Esters | 3.00 |
| PHYTOSQUALAN | Squalene | 7.00 |
| BERNEL ESTER 105 | Isodecyl Neopentanoate | 5.00 |
| PRESERVATIVE | | Q.S. |
| DERMOFEEL TOCO 70 | Tocopherol (and) Helianthuus Annus (sunflower) Seed Oil | 0.05 |
| PHYTOWAX OLIVE 18L57 | Hydrogenated Stearyl Olive Esters | 2.00 |
| OPTISOL | Titanium Dioxide | 0.3 |
| | | |

| **PHASE B** | | |
|---|---|---|
| WATER | AQUA | Q.S. to 100 |
| ZEMEA | Propanediol | 10.00 |
| BIOCOL AX | Pectin (and) Xanthan gum | 1.00 |
| XANTHAN GUM | Xanthan Gum | 0.35 |
| | | |

| **PHASE C** | | |
|---|---|---|
| SENSHYAL | Hyaluronic Acid (and) Rhizobian Gum | 2.00 |
| HYDROXAN CH | Glycerin (and) Propylene Glycol (and) Sorbitol (and) Carboxymethylchitin (and) Panthenol (and) Sodium Hyaluronate | 1.00 |
| HOMEOXY | Sorbitol (and) Enteromorpha Compressa Extract (and) Palmaria Palmata Extract (and) Water | 1.00 |
| MDI COMPLEX | Glycosaminoglycans | 2.00 |
| HYDRIAME | Water (and) Glycosaminoglycans (and) Sclerotium Gum (and) Chitosan Glycolate (and) Sodium Hydroxide | 1.00 |
| LANABLUE | Sorbitol (and) Water (and) Algae Extract | 1.50 |
| NEUTRAZEN | Water (and) Butylene glycol (and) Dextran (and) Palmitoyl Tripeptide-8 | 0.50 |
| EXO H | Butylene Glycol (and) Alteromonas Ferment Filtrate | 1.50 |
| RED CORAL | Coral Powder | 5.00 |
| KSG-15 | Dimethicone/Vinyldimethicone Crosspolymer | 2.00 |
| FRAGRANCE | Parfum | Q.S. |
| GLUCONIC ACID | Gluconic Acid | Q.S. to pH 6.2 |

This cream is prepared as follows.

The ingredients listed under Phase A are mixed and heated to 75°C.

Phase B is prepared by heating propanediol and water to 75°C and by dispersing thereinto BIOCOL AX and Xanthan gum.

Phase A is added to Phase B and the resulting mixture is stirred in a turbo-mixer for 10 minutes. Afterwards, the mixture is cooled to 40°C under fast stirring. Once this temperature has been reached, all the components listed under Phase C, but gluconic acid, are added one after the other to the above mixture and the final mixture is brought under slow stirring down to room temperature. Finally, gluconic acid is added in the required amount to adjust the pH to 6.2.

### Example 7

**EXFOLIATING FACE GEL**

| **PHASE A** | | |
|---|---|---|
| DERMOL 816 | Ethylhexyl Palmitate | 26.8 |
| TRIFAT S 308 | Triethylhexanoin | 10.4 |
| DERMOL JOBA | Cetearyl Ethylhexanoate | 9.4 |
| DEXTRIN PALMITATE | Dextrin Palmitate | 5.6 |
| | | |

| **PHASE B** | | |
|---|---|---|
| TGI-20 | PEG-20 Glyceryl Triisostearate | 14.15 |
| PHYTOSQUALAN | Squalane | 18.9 |
| | | |
| KSG-43 | Dimethicone/Vinyldimethicone Crosspolymer | 2.8 |
| TRIFAT S 308 | Triethylhexanoin | 6.6 |
| | | |

| **PHASE C** | | |
|---|---|---|
| PRESERVATIVE | | Q.S. |
| RED CORAL | Coral Powder | 1.50 |
| OYSTER SHELL | Oyster Shell Powder | 3.00 |
| FRAGRANCE | Parfum | Q.S. |

This cream is prepared as follows.

The first three ingredients listed under Phase A are mixed and heated to 105°C and DEXTRIN PALMITATE is slowly added thereto.

TGI-20 and PHYTOSQUALAN are added to Phase A, after the latter has been brought to 80°C. KSG-43 is dispersed into TRIFAT S 308 and the resulting mixture is added, at 80°C, to the mixture of Phase A, TGI-20 and PHYTOSQUALAN. Then, the resulting mixture is brought to a temperature of 40°C and the components of Phase C are added thereto.

### Example 8

**FACE POWDER**

| | | |
|---|---|---|
| RED CORAL | Coral Powder | 99.0 |
| FRAGRANCE | Parfum | Q.S. |
| PRESERVATIVE | | Q.S. |

### Example 9

**ANTI-AGEING "COUP D'ECLAT" FACE CREAM**

| **PHASE A** | | |
|---|---|---|
| EMULPROGRESS 57 | Polyglyceryl-10 Stearate (and) Polyglyceryl-6 Tristearate (and) Cetearyl Alcohol (and) Hydroxypropyl Guar | 4.00 |
| PHYTOSQUALAN | Squalene | 7.00 |
| BERNEL ESTER 105 | Isodecyl Neopentanoate | 5.00 |
| PRESERVATIVE | | Q.S. |
| DERMOFEEL TOCO 70 | Tocopherol (and) Helianthuus Annus (sunflower) Seed Oil | 0.05 |
| OPTISOL | Titanium Dioxide | 0.3 |

| **PHASE B** | | |
|---|---|---|
| WATER | AQUA | Q.S. to 100 |
| ZEMEA | Propanediol | 10.00 |
| | | |
| | | |
| | | |

| **PHASE C** | | |
|---|---|---|
| SENSHYAL | Hyaluronic Acid (and) Rhizobian Gum | 2.00 |
| HYDROXAN CH | Glycerin (and) Propylene Glycol (and) Sorbitol (and) Carboxymethylchitin (and) Panthenol (and) Sodium Hyaluronate | 1,00 |
| HOMEOXY | Sorbitol (and) Enteromorpha Compressa Extract (and) Palmaria Palmata Extract (and) Water | 1.00 |
| MDI COMPLEX | Glycosaminoglycans | 2.00 |
| HYDRIAME | Water (and) Glycosaminoglycans (and) Sclerotium Gum (and) Chitosan Glycolate (and) Sodium Hydroxide | 1.00 |
| LANABLUE | Sorbitol (and) Water (and) Algae Extract | 1.50 |
| NEUTRAZEN | Water (and) Butylene glycol (and) Dextran (and) Palmitoyl Tripeptide-8 | 0.50 |
| EXO H | Butylene Glycol (and) Alteromonas Ferment Filtrate | 1.50 |
| RED CORAL | Coral Powder | 5.00 |
| KSG-15 | Dimethicone/Vinyldimethicone Crosspolymer | 2.00 |
| FRAGRANCE | Parfum | Q.S. |
| LACTIC ACID | Lactic Acid | Q.S. to pH 6.2 |

This cream is prepared as follows.

The ingredients listed under Phase A are mixed and heated to 75°C.

Phase B is prepared by heating to 75°C

Phase A is added to Phase B and the resulting mixture is stirred in a turbo-mixer for 10 minutes. Afterwards, the mixture is cooled to 40°C under fast stirring. Once this temperature has been reached, all the components listed under Phase C, but lactic acid, are added one after the other to the above mixture and the final mixture is brought under slow stirring down to room temperature. Finally, lactic acid is added in the required amount to adjust the pH to 6.2.

### Example 10

**RESTITUTIVE ANTI-AGEING FACE CREAM**

| **PHASE A** | | |
|---|---|---|
| EMULPROGRESS 57 | Polygryceryl-10 Stearate (and) Polyglyceryl-6 Tristearate (and) Cetearyl Alcohol (and) Hydroxypropyl Guar | 6.00 |
| BIOPHYTOSEBUM | Decyl Olive Esters (and) Squalene | 5.00 |
| PHYTOSQUALAN | Squalene | 3.00 |
| OLIVE CERESTERS | Decyl Olivate | 2.00 |
| PRESERVATIVE | | Q.S. |
| DERMOFEEL TOCO 70 | Tocopherol (and) Helianthuus Annus (sunflower) Seed Oil | 0.05 |
| PHYTOWAX OLIVE 18L57 | Hydrogenated Stearyl Olive Esters | 1.50 |

| **PHASE B** | | |
|---|---|---|
| WATER | AQUA | Q.S. to 100 |
| ZEMEA | Propanediol | 5.00 |
| BIOCOL AX | Pectin (and) Xanthan gum | 1.00 |

| **PHASE C** | | |
|---|---|---|
| SENSHYAL | Hyaluronic Acid (and) Rhizobian Gum | 2.00 |
| OLIGOGELINE | Condrus Crispus (Carraggenan) | 2.00 |
| HOMEO-AGE | Ascophyllum Nodosum Extract (and) Sorbitol (and) Water | 1.50 |
| HOMEOXY | Sorbitol (and) Enteromorpha Compressa Extract (and) Palmaria Palmata Extract (and) Water | 1.00 |
| CHRONOLINE | Glycerin (and) Water (and) Dextran (and) Caprooyl-Tetrapeptide-3 | 1.00 |
| KOLLAREN | Water (and) Dextran (and) Tripeptide-1 | 3.50 |
| HOMEO-SHIELD | Fucus Serratus Extract (and) Glycerin | 1.00 |
| HYDRIAME | Water (and) Glycosaminoglycans (and) Sclerotium Gum (and) Chitosan Glycolate (and) Sodium Hydroxide | 3.00 |
| LANABLUE | Sorbitol (and) Water (and) Algae Extract | 2.50 |
| NEUTRAZEN | Water (and) Butylene glycol (and) Dextran (and) Palmitoyl Tripeptide-8 | 1.00 |
| EXO T | Butylene Glycol (and) Vibrio Alginolyticus Ferment Filtrate | 0.50 |
| EXO H | Butylene Glycol (and) Alteromonas Ferment Filtrate | 1.00 |
| RED CORAL | Coral Powder | 2.00 |
| TRANSCUTOL CG | Ethoxydiglycol | 1.20 |
| KSG-15 | Dimethicone/Vinyldimethicone Crosspolymer | 1.50 |
| PHYLCARE SODIO IALURONATO EXTRA LW | SODIUM HYALURONATE | 0.10 |
| PHYLCARE SODIO IALURONATO EXTRA HW | SODIUM HYALURONATE | 0.10 |
| FRAGRANCE | Parfum | Q.S |
| LACTIC ACID | Lactic Acid | Q.S. to pH 5.8 |

This cream was prepared in the same way as for the cream of Example 5.

### Example 11

**ANTI-AGEING ENLIGHTENING FACE CREAM**

| **PHASE A** | | |
|---|---|---|
| EMULIUM 22 | Tribehenin PEG-20 Esters | 3.00 |
| PHYTOSQUALAN | Squalene | 7.00 |
| BERNEL ESTER 105 | Isodecyl Neopentanoate | 5.00 |
| PRESERVATIVE | | Q.S. |
| DERMOFEEL TOCO 70 | Tocopherol (and) Helianthuus Annus (sunflower) Seed Oil | 0.05 |
| PHYTOWAX OLIVE 18L57 | Hydrogenated Stearyl Olive Esters | 2.00 |
| OPTISOL | Titanium Dioxide | 0.3 |
| | | |

| **PHASE B** | | |
|---|---|---|
| WATER | AQUA | Q.S. to 100 |
| ZEMEA | Propanediol | 10.00 |
| BIOCOL AX | Pectin (and) Xanthan gum | 1.00 |
| XANTHAN GUM | Xanthan Gum | 0.35 |

| **PHASE C** | | |
|---|---|---|
| SENSHYAL | Hyaluronic Acid (and) Rhizobian Gum | 2.00 |
| HYDROXAN CH | Glycerin (and) Propylene Glycol (and) Sorbitol (and) Carboxymethylchitin (and) Panthenol (and) Sodium Hyaluronate | 1.00 |
| HOMEOXY | Sorbitol (and) Enteromorpha Compressa Extract (and) Palmaria Palmata Extract (and) Water | 1.00 |
| MDI COMPLEX | Glycosaminoglycans | 2.00 |
| HYDRIAME | Water (and) Glycosaminoglycans (and) Sclerotium Gum (and) Chitosan Glycolate (and) Sodium Hydroxide | 1.00 |
| LANABLUE | Sorbitol (and) Water (and) Algae Extract | 1.50 |
| NEUTRAZEN | Water (and) Butylene glycol (and) Dextran (and) Palmitoyl Tripeptide-8 | 0.50 |
| EXO H | Butylene Glycol (and) Alteromonas Ferment Filtrate | 1.50 |
| RED CORAL | Coral Powder | 5.00 |
| KSG-15 | Dimethicone/Vinyldimethicone Crosspolymer | 2.00 |
| PHYLCARE SODIO IALURONATO EXTRA LW | SODIUM HYALURONATE | 0.10 |
| PHYLCARE SODIO IALURONATO EXTRA HW | SODIUM HYALURONATE | 0.10 |
| FRAGRANCE | Parfum | Q.S. |
| GLUCONIC ACID | Gluconic Acid | Q.S. to pH 6.2 |

This cream was prepared in the same way as for the cream of Example 6.

### Example 12

**ANTI-AGEING "COUP D'ECLAT" FACE CREAM**

| **PHASE A** | | |
|---|---|---|
| EMULPROGRESS 57 | Polyglyceryl-10 Stearate (and) Polyglyceryl-6 Tristearate (and) Cetearyl Alcohol (and) Hydroxypropyl Guar | 4.00 |
| PHYTOSQUALAN | Squalene | 7.00 |
| BERNEL ESTER 105 | Isodecyl Neopentanoate | 5.00 |
| PRESERVATIVE | | Q.S. |
| DERMOFEEL TOCO 70 | Tocopherol (and) Helianthuus Annus (sunflower) Seed Oil | 0.05 |
| OPTISOL | Titanium Dioxide | 0.3 |
| | | |

| **PHASE B** | | |
|---|---|---|
| WATER | AQUA | Q.S. to 100 |
| ZEMEA | Propanediol | 10.00 |
| | | |
| | | |
| | | |

| **PHASE C** | | |
|---|---|---|
| SENSHYAL | Hyaluronic Acid (and) Rhizobian Gum | 2.00 |
| HYDROXAN CH | Glycerin (and) Propylene Glycol (and) Sorbitol (and) Carboxymethylchitin (and) Panthenol (and) Sodium Hyaluronate | 1,00 |
| HOMEOXY | Sorbitol (and) Enteromorpha Compressa Extract (and) Palmaria Palmata Extract (and) Water | 1.00 |
| MDI COMPLEX | Glycosaminoglycans | 2.00 |
| HYDRIAME | Water (and) Glycosaminoglycans (and) Sclerotium Gum (and) Chitosan Glycolate (and) Sodium Hydroxide | 1.00 |
| LANABLUE | Sorbitol (and) Water (and) Algae Extract | 1.50 |
| NEUTRAZEN | Water (and) Butylene glycol (and) Dextran (and) Palmitoyl Tripeptide-8 | 0.50 |
| EXO H | Butylene Glycol (and) Alteromonas Ferment Filtrate | 1.50 |
| RED CORAL | Coral Powder | 5.00 |
| KSG-15 | Dimethicone/Vinyldimethicone Crosspolymer | 2.00 |
| PHYLCARE SODIO IALURONATO EXTRA LW | SODIUM HYALURONATE | 0.10 |
| PHYLCARE SODIO IALURONATO EXTRA HW | SODIUM HYALURONATE | 0.10 |
| FRAGRANCE | Parfum | Q.S. |
| LACTIC ACID | Lactic Acid | Q.S. to pH 6.2 |

This cream was prepared in the same way as for the cream of Example 9.

## Claims

1. A cosmetic formulation for application to the skin, comprising a powder obtained from the milling of red coral (Corallium rubrum) and optionally a cosmetically acceptable vehicle, wherein said powder has a particle size comprised between 4 and 20 µm and has a pale pink to intense pink color, for use as skin care, make-up and/or sun tanning product.

2. A cosmetic formulation according to claim 1 endowed with anti-ageing function, wherein said powder has a particle size of about 4-6 µm" preferably 4 to less than 5 µm.

3. A cosmetic formulation according to claim 1, with soft focus agent and radiance enhancer function, wherein said powder has a particle size comprised between 8 and 20 µm.

4. A cosmetic formulation according to any one of claims 1 to 3, containing 0.1% to 100% red coral (Corallium rubrum) powder by weight out of the total weight of the formulation.

5. A cosmetic formulation according to claim 4, containing 95% to 100% red coral (Corallum rubrum) powder by weight out of the total weight of the formulation, for use as a face powder or blush.

6. A cosmetic formulation according to claim 4, containing 0.1 to 10%, preferably 0.25% to 2%, red coral (Corallium rubrum) powder by weight out of the total weight of the formulation together with a cosmetically acceptable vehicle and optionally at least one additional cosmetically active ingredient.

7. A cosmetic formulation according to claim 6, further containing at least one weak acid selected among lower carboxylic acids and phosphoric acid.

8. A cosmetic formulation according to claim 7, wherein said at least one weak acid is lactic acid or gluconic acid and is contained in an amount of about 0.1% to 10% by weight out of the weight of the composition.

9. Use of red coral (Corallium rubrum) powder as a cosmetically active substance for application to the skin, wherein said red coral powder has a particle size comprised between 4 and 300 µm and has a pale pink to intense pink color.

10. A method of cosmetic treatment, which comprises the step of applying onto the skin red coral (Corallium rubrum) powder or a cosmetic formulation containing it, wherein said red coral powder has a particle size comprised between 4 and 300 µm and has a pale pink to intense pink color.

## Patentansprüche

1. Kosmetische Formulierung zur Anwendung auf der Haut, die ein Pulver, erhalten aus dem Zermahlen von roten Korallen (Corallium rubrum) und gegebenenfalls einen kosmetisch verträglichen Träger, enthält, wobei das Pulver eine Partikelgröße zwischen 4 und 20 µm aufweist und eine hellrosa bis intensivrosa Farbe hat, zur Verwendung als Hautpflege, Make-up und/oder Sonnenbräunungsprodukt.

2. Kosmetische Formulierung gemäß Anspruch 1, die mit einer Antialterungsfunktion versehen ist, wobei das Pulver eine Partikelgröße von etwa 4-6 µm, vorzugsweise von 4 bis weniger als 5µm, aufweist.

3. Kosmetische Formulierung gemäß Anspruch 1, mit Weichzeichnungsmittel und Glanzsteigerungsfunktion, wobei das Pulver eine Teilchengröße zwischen 8 und 20 µm aufweist.

4. Kosmetische Formulierung gemäß einem der Ansprüche 1 bis 3, enthaltend 0,1 % bis 100 % des Gesamtgewichts der Formulierung an Pulver der roten Koralle (Corallium rubrum).

5. Kosmetische Formulierung gemäß Anspruch 4, enthaltend 95 bis 100 % des Gesamtgewichts der Formulierung an Pulver der roten Koralle (Corallium rubrum) für die Verwendung als Gesichtspuder oder Wangenrot.

6. Kosmetische Formulierung gemäß Anspruch 4, enthaltend 0,1 bis 10%, vorzugsweise 0,25 bis 2 %, des Gesamtgewichts der Formulierung an Pulver der roten Koralle (Corallium rubrum) zusammen mit einem kosmetisch verträglichen Träger und gegebenenfalls mindestens einem zusätzlichen kosmetisch aktiven Bestandteil.

7. Kosmetische Formulierung nach Anspruch 6, die ferner mindestens eine schwache Säure enthält, ausgewählt aus niederen Carbonsäuren und Phosphorsäure.

8. Kosmetische Formulierung nach Anspruch 7, wobei die mindestens eine schwache Säure Milchsäure oder Gluconsäure ist und in einer Menge von etwa 0,1% bis 10 Gewichts-% bezogen auf das Gewicht der Zusammensetzung enthalten ist.

9. Verwendung von Pulver aus roten Korallen (Corallium rubrum) als kosmetisch aktive Substanz für die Anwendung auf der Haut, wobei das Pulver der roten Koralle eine Partikelgröße zwischen 4 und 300 µm aufweist und eine hellrosa bis intensivrosa Farbe hat.

10. Verfahren zur kosmetischen Behandlung umfassend den Schritt des Auftragens auf die Haut von Pulver aus roter Koralle (Corallium rubrum) oder einer kosmetischen Formulierung, die sie enthält, wobei das Pulver der roten Koralle eine Partikelgröße zwischen 4 und 300 µm aufweist und eine hellrosa bis intensivrosa Farbe hat.

## Revendications

1. Formulation cosmétique à appliquer sur la peau, comprenant une poudre obtenue par broyage de corail rouge (Corallium rubrum) et éventuellement un véhicule compatible du point de vue cosmétique, dans laquelle la poudre a une granulométrie comprise entre 4 et 20 µm, et a une couleur rose pâle à rose intense, pour une utilisation pour le soin de la peau, le maquillage et/ou comme produit solaire.

2. Formulation cosmétique selon la revendication 1, dotée d'une fonction anti-vieillissement, la poudre ayant une granulométrie d'environ 4-6 µm, de préférence de 4 à moins de 5 µm.

3. Formulation cosmétique selon la revendication 1, avec un agent de floutage (soft focus) et la fonction d'activateur d'éclat, la poudre présentant une granulométrie comprise entre 8 et 20 µm.

4. Formulation cosmétique selon l'une quelconque des revendications 1 à 3, contenant 0,1 % à 100 % de poudre de corail rouge (Corallium rubrum), pourcentages rapportés au poids total de la formulation.

5. Formulation cosmétique selon la revendication 4, contenant 95 % à 100 % de poudre de corail rouge (Corallium rubrum), pourcentages rapportés au poids total de la formulation, pour une utilisation en tant que poudre pour le visage ou de fard à joues.

6. Formulation cosmétique selon la revendication 4, contenant 0,1 à 10 %, de préférence de 0,25 % à 2 %, de poudre de corail rouge (Corallium rubrum), pourcentages rapportés au poids total de la formulation, conjointement avec un véhicule compatible du point de vue cosmétique et éventuellement au moins un autre ingrédient actif du point de vue cosmétique.

7. Formulation cosmétique selon la revendication 6, contenant en outre au moins un acide faible choisi parmi les acides carboxyliques de faible masse moléculaire et l'acide phosphorique.

8. Formulation cosmétique selon la revendication 7, dans lequel ledit au moins un acide faible est l'acide lactique ou l'acide gluconique, et il est présent en une quantité d'environ 0,1 % à 10 % en poids rapportés au poids de la composition.

9. Utilisation de poudre de corail rouge (Corallium rubrum) comme substance active du point de vue cosmétique pour une application sur la peau, la poudre de corail rouge ayant une granulométrie comprise entre 4 et 300 µm et une couleur rose pâle à rose intense.

10. Procédé de traitement cosmétique qui comprend l'étape consistant à appliquer sur la peau la poudre de corail rouge (Corallium rubrum) ou une formulation cosmétique qui en contient, la poudre de corail rouge ayant une granulométrie comprise entre 4 et 300 µm et une couleur rose pâle à rose intense.
